# EUROPEAN PATENT APPLICATION

(11) **EP 2 016 941 A1**
(43) Date of publication of application: **21.01.2009**
(21) Application number: 07742960.3
(22) Date of filing: 08.05.2007
(51) Int. Cl.: A61K 31/445, A61K 9/70, A61K 47/32, A61P 25/28, C07D 211/32

(54) **TRANSDERMALLY ABSORBABLE DONEPEZIL PREPARATION**

(30) Priority: 09.05.2006 JP 2006130668
(71) Applicant: Hisamitsu Pharmaceutical Co., Inc., Tosu-shi, Saga 841-0017 (JP)
(72) Inventor: TERAHARA, Takaaki, Tsukuba-shi, Ibaraki 305-0856 (JP); MICHINAKA, Yasunari, Tsukuba-shi, Ibaraki 305-0856 (JP); NAKANISHI, Masaru, Tsukuba-shi, Ibaraki 305-0856 (JP); HATTORI, Wataru, Tsukuba-shi, Ibaraki 305-0856 (JP); KURODA, Takao, Tokyo 100-6221 (JP); AIDA, Kazunosuke, Tsukuba-shi, Ibaraki 305-0856 (JP)
(74) Representative: Adam, Holger
(86) International application number: PCT/JP2007/059525
(87) International publication number: WO 2007/129712

(57) **Abstract**

Disclosed is a donepezil-containing transdermally absorbable preparation which develops reduced adverse side effects and shows a satisfactory level of therapeutic effect. The preparation comprises an adhesive and a donepezil component (containing crystalline donepezil having type-B crystal polymorphism) and/or a salt thereof, wherein the donepezil component or the salt thereof is contained in an amount of 9 to 50% by mass relative to the total weight of the adhesive. The preparation (particularly, one having a non-aqueous adhesive layer) shows an excellent penetration of donepezil and/or a salt thereof into the skin, retains good stability of the active ingredient therein, and is remarkably reduced in local stimulation and adverse side effects.

## Description

### Technical Field

The present invention relates to a transdermal preparation comprising donepezil and/or a chemically acceptable salt thereof, which is known as a therapeutic agent for Alzheimer type dementia, that is capable of developing remarkably reduced side effects, and a method of administration to reduce side effects in donepezil therapy. More particularly, the present invention relates to a donepezil-containing transdermal preparation for the treatment of Alzheimer type dementia, Down syndrome and attention-deficit hyperactivity disorder, which has remarkably reduced side effects by reducing the fluctuation of the plasma concentration of donepezil itself, as well as by suppressing generation of the metabolites of donepezil and/or a chemically acceptable salt thereof, and a method of administration to reduce side effects in donepezil therapy.

### Background Art

As for the drug therapy for Alzheimer type dementia, an acetylcholinesterase inhibitor such as donepezil is effective. However, donepezil that can be actually used at present is being formulated into oral preparations, and under current circumstances, such preparations have problems of a transient rapid rise of blood concentration, or side effects caused by the drug's direct action on the gastrointestinal tract, metabolites and the like. Furthermore, since Alzheimer type dementia patients who are generally anticipated to benefit from the therapeutic effects of donepezil are often elderly persons who suffer from deterioration in the functions of the digestive system, it is difficult to retain a constant bioavailability of oral administration, and in many cases, there occur problems with compliance as well. In addition, most of the patients of Down syndrome and attention-deficit hyperactivity disorder who are anticipated to benefit from the therapeutic effects of donepezil are children, and may develop serious side effects in some cases, and likewise in the case of elderly persons, it is feared that there are many cases where compliance becomes a problem. The donepezil preparations currently used are oral preparations such as tablets or orally disintegrating tablets containing hydrochloride salt, and these preparations are not capable of avoiding metabolism and degradation in the liver, and are likely to exhibit side effects in the digestive system. Particularly, in order to avoid the side effects caused by the fluctuation of bioavailability and the transient rapid rise in the blood concentration, in the case of orally administering donepezil hydrochloride, it is necessary to perform administration while gradually increasing the daily dosage. Directions are given by physicians such that in Japan, 3 mg is administered once daily up to 1 to 2 weeks after the initial administration, and then the dose is increased to 5 mg, while in countries other than Japan, 5 mg is administered once daily up to 4 to 6 weeks after the initial administration, and then the dose is increased to 10 mg. In order to avoid such complicated operation of setting the dosage regimen for the orally administered preparations, and also to allow reduction in side effects by suppressing a transient rapid rise of the blood concentration, several transdermal preparations comprising a donepezil derivative have been suggested in recent years, as preparations substituting oral preparations.

There are cases where intake of oral preparations becomes difficult, for example, such as dementia patients showing advanced symptoms, and as a dosage form appropriate for administration in such cases, ointments and the like for transdermal administration, and suppositories for rectal administration have been proposed (Patent Document 1). Said document also shows that transdermal absorbability of donepezil hydrochloride is enhanced by a base containing a higher alcohol and an ester derivative thereof. However, although examples of using ointments, creams and suppositories may be mentioned in this document as Examples, these preparations are not practical for continuously administering the active ingredient over a long time. Furthermore, there also has been proposed a transdermal preparation for anti-Alzheimer type dementia drugs containing a basic inorganic substance as an absorption enhancer (Patent Document 2).

However, in any of the above-mentioned documents, the methods for reducing side effects mainly depend on the change of dosage form from oral preparations to transdermal preparations or suppositories, and thus the methods lack specificity. In addition, a tape preparation for anti-Alzheimer type dementia drugs has also been proposed (Patent Document 3). The said document also suggests the necessary drug absorption rate, changes in the blood concentration, and the like, but does not describe on a method for reducing side effects. Moreover, a method and a transdermal preparation for reducing side effects of oxybutynin, which is a therapeutic drug for frequent urination (Patent Document 4), a method and a transdermal preparation for reducing side effects of pergolide, which is a therapeutic drug for Parkinson's disease (Patent Document 5), and the like have also been proposed. However, in these documents, nothing is mentioned at all on a transdermal preparation comprising a donepezil derivative, as well as on a therapeutic drug for Alzheimer type dementia, and moreover, the said documents do not offer any specific opinion on the reduction of side effects caused by a donepezil derivative, or the like.

That is to say, attempts have been made to avoid the influence of metabolism by employing a transdermally absorbable dosage form, thereby allowing donepezil to show sufficient efficacy, however, a transdermal preparation suppressing side effects at the same time, or a method for administration thereof is not known at all. For that reason, it is desired to develop a transdermal preparation which overcomes the above-described problems in donepezil therapy and is capable of reducing side effects, and a method for administration thereof.
[Patent Document 1] JP-A No. 11-315016
[Patent Document 2] U.S. Patent No. 6815454 B2
[Patent Document 3] WO 2003/032960
[Patent Document 4] JP-W No. 2003-531157
[Patent Document 5] WO 2005/041967

### Disclosure of the Invention

### Problems to be Solved by the Invention

It is an object of the present invention to provide a donepezil derivative-containing transdermal preparation which allows reduction of side effects and exhibits satisfactory therapeutic effects.

### Means for Solving the Problems

The inventors of the present invention devotedly conducted investigation to solve the above-described problems in the related art, and as a result, found that a transdermal preparation comprising crystalline donepezil having type-B crystal polymorphism and/or a salt thereof in a certain amount or more, has excellent skin permeability, excellent persistence of the plasma concentration of donepezil, and excellent suppressive ability on side effects. Thus, the inventors completed the following various inventions.

(1) A transdermal preparation which comprises donepezil including crystalline donepezil having type-B crystal polymorphism and/or a salt thereof, in an amount of 9% by mass to 50% by mass based on the total weight of an adhesive.

(2) The transdermal preparation according to (1), characterized in that the donepezil including crystalline donepezil having type-B crystal polymorphism and/or a chemically acceptable salt thereof is donepezil base.

(3) The transdermal preparation according to (1) or (2), wherein at least 30% or more of the donepezil and/or salt thereof contained in an adhesive layer is crystalline donepezil having type-B crystal polymorphism.

(4) The transdermal preparation according to any one of (1) to (3), wherein the preparation does not essentially contain water.

(5) The transdermal preparation according to any one of (1) to (4), which comprises at least one adhesive selected from an acrylic polymer and a rubber-based polymer in the adhesive.

(6) The transdermal preparation according to (5), wherein the acrylic polymer is a copolymer containing 2-ethylhexyl acrylate.

(7) The transdermal preparation according to (5), wherein the rubber-based polymer is one or more selected from the group consisting of polyisobutylene, styrene-isoprene-styrene block copolymers and silicone adhesives.

(8) The transdermal preparation according to any one of (1) to (5), wherein the ratio of the maximum plasma concentration (A) and the minimum plasma concentration (B) (A/B, peak-trough ratio) of donepezil and/or a chemically acceptable salt thereof after administration is 1.5 or less.

(9) The transdermal preparation according to any one of (1) to (5), yielding at least one or more of 5-O-desmethyldonepezil, 6-O-desmethyldonepezil and donepezil-cis-N-oxide, as the metabolite of donepezil and/or a chemically acceptable salt thereof in the plasma after administration.

(10) The transdermal preparation according to (9), wherein the amount of generation in the plasma for one kind of the metabolite is less than 5% of the amount of donepezil and/or a chemically acceptable salt thereof contained in the transdermal preparation.

(11) The transdermal preparation according to (9) or (10), wherein the AUC of the metabolite of donepezil after the administration of donepezil and/or a chemically acceptable salt thereof is 20% or less of the AUC of donepezil and/or a chemically acceptable salt thereof.

(12) The transdermal preparation according to (11), wherein the AUC of the metabolite of donepezil for up to 12 hours after administration is less than 50 ng/mL.hr.

(13) The transdermal preparation according to any one of (1) to (5), wherein the lag time associated with the absorption of donepezil and/or a chemically acceptable salt thereof is 3 hours or longer.

(14) A method for administering the transdermal preparation according to any one of (1) to (5), wherein the maximum absorption rate of donepezil and/or a chemically acceptable salt thereof is less than 1700 µg/hr.

### Effects of the Invention

By applying such transdermal preparation comprising donepezil including crystalline donepezil having type-B crystal polymorphism and/or a chemically acceptable salt thereof (particularly, containing the component in a non-aqueous adhesive layer), a transdermal preparation having very good skin permeability and good drug stability in the preparation, while having less localized stimulation can be obtained.

### Best Mode for Carrying Out the Invention

The present invention relates to a transdermal preparation which contains donepezil including crystalline donepezil having type-B crystal polymorphism and/or a chemically acceptable salt thereof.

The crystal polymorphism of donepezil is known to include type A, type B and type C (WO 99/29668 pamphlet). The inventors of the present invention unexpectedly discovered that by controlling the content of crystalline donepezil having type-B crystal polymorphism in an adhesive layer, the transdermal absorbability of donepezil can be enhanced, while suppressing theincidence of metabolites in the body after administration, so as to reduce side effects.

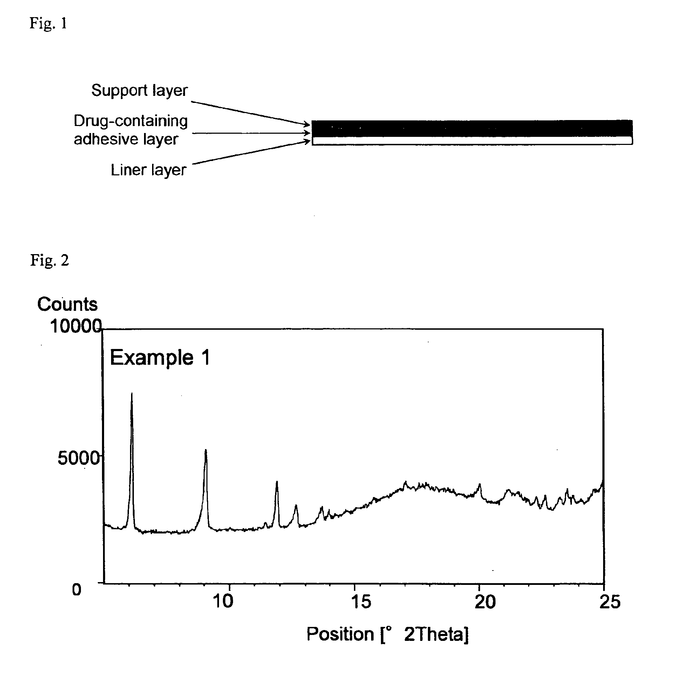

The dosage form of the transdermal preparation of the present invention is not particularly limited, but for example, it is preferred an adhesive patch having the configuration shown in Fig. 1, while an adhesive patch containing substantially no water is particularly preferred.

The chemically acceptable salt that can be used in the present invention is not particularly limited, and may be an inorganic salt or an organic salt. As for the inorganic salt, salts of hydrochloric acid, hydrobromic acid, silicic acid and the like may be mentioned, and in particular, hydrochloric acid salt is preferred. As for the organic salt, salts of acetic acid, citric acid, fumaric acid, maleic acid and the like may be mentioned, and in particular, acetic acid salt is preferred.

It is preferable that the transdermal preparation of the present invention comprises donepezil including crystalline donepezil having type-B crystal polymorphism and/or a chemically acceptable salt thereof in an amount of 9% by mass to 50% by mass, preferably 9% by mass to 30% by mass, based on the total weight of an adhesive. Furthermore, the donepezil and/or chemically acceptable salt thereof in the adhesive layer may be a mixture of soluble donepezil (S) and crystalline donepezil (C) having type-B crystal polymorphism, but the content of the crystalline donepezil (C) in the total amount of donepezil and/or a chemically acceptable salt thereof in the adhesive layer is at least 30% or greater, preferably 50% or greater, and particularly preferably 90% or greater. Furthermore, the crystalline donepezil is preferably a type-B crystal polymorphism of donepezil base.

When the dosage form of the present invention is an adhesive patch, it is preferable to use an acrylic polymer or a rubber-based polymer as the base of the adhesive layer. The acrylic polymer is not particularly limited as long as it contains a copolymer comprising at least one of (meth)acrylic acid derivatives which are represented by 2-ethylhexyl acrylate, methyl acrylate, butyl acrylate, hydroxyethyl acrylate, 2-ethylhexyl methacrylate and the like, but preferably, it is desirable that the acrylic polymer contain 2-ethylhexyl acrylate in an amount of 50% or more.

Specific examples of the adhesive that can be used include adhesives such as acrylic acid/acrylic acid octyl ester copolymers, 2-ethylhexyl acrylate/vinylpyrrolidone copolymer solutions, acrylic acid ester/vinyl acetate copolymers, 2-ethylhexyl acrylate/2-ethylhexyl methacrylate/dodecyl methacrylate copolymers, methyl acrylate/2-ethylhexyl acrylate copolymerized resin emulsions, and acrylic polymers contained in acrylic resin alkanolamine solutions, DURO-TAK acrylic adhesive series (National Starch and Chemical Company), Eudragit series (Higuchi, Inc.), and the like.

As for the rubber-based copolymer, a styrene-isoprene-styrene block copolymer (hereinafter, abbreviated to SIS), an isoprene rubber, polyisobutylene (hereinafter, abbreviated to PIB), a styrene-butadiene-styrene block copolymer (hereinafter, abbreviated to SBS), a styrene-butadiene rubber (hereinafter, abbreviated to SBR), polysiloxane, and the like may be mentioned, and among them, SIS, PIB or polysiloxanes is preferred, and SIS and PIB are particularly preferred.

Such hydrophobic polymers may be used as a mixture of two or more kinds, and the additive amount of these polymers based on the weight of the adhesive layer is preferably 5 to 90% by mass, more preferably 10 to 70% by mass, and particularly preferably 10 to 50% by mass, in consideration of the formation of an adhesive layer and sufficient permeability.

The adhesive layer in the preparation of the present invention may contain a plasticizer. As for the plasticizer that can be used, petroleum-based oils (for example, paraffinic process oils, naphthenic process oils, aromatic process oils, and the like), squalane, squalene, plant oils (for example, olive oil, camellia oil, castor oil, tall oil, peanut oil), silicone oils, dibasic acid esters (for example, dibutyl phthalate, dioctyl phthalate, and the like), liquid rubbers (for example, polybutene, liquid isoprene rubber), liquid fatty acid esters (isopropyl myristate, hexyl laurate, diethyl sebacate, diisopropyl sebacate), diethylene glycol, polyethylene glycol, glycol salicylate, propylene glycol, dipropylene glycol, triacetin, triethyl citrate, crotamiton and the like may be mentioned. Among them, liquid paraffin, liquid polybutene, isopropyl myristate, diethyl sebacate, and hexyl laurate are preferred, and liquid polybutene, isopropyl myristate and liquid paraffin are particularly preferred. These components may be used as mixtures of two or more kinds.

In the case of adding a plasticizer, the additive amount thereof based on the weight of the adhesive layer is 10 to 70% by mass, preferably 10 to 60% by mass, and more preferably 10 to 50% by mass, in view of the maintenance of sufficient permeability and sufficient cohesive force required from a patch preparation.

In the case where the present invention is an adhesive patch, the adhesive layer preferably contains a tackifier resin if the adhesive force is insufficient, and examples of the tackifier resin that can be used include rosin derivatives (for example, rosins, glycerin esters of rosins, hydrogenated rosins, glycerin esters of hydrogenated rosins, pentaerythritol esters of rosins, and the like), alicyclic saturated hydrocarbon resins (for example, Arkon P100, Arakawa Chemical Industries, Ltd.), aliphatic hydrocarbon resins (for example, Quinton B 170, Nippon Zeon Co., Ltd.), terpene resins (for example, Clearon P-125, Yasuhara Chemical Co., Ltd.), maleic acid resins and the like. In particular, glycerin esters of hydrogenated rosins, alicyclic saturated hydrocarbon resins, aliphatic hydrocarbon resins, and terpene resins are preferred.

In the case of adding a tackifier resin, the additive amount based on the weight of the adhesive layer is 5 to 70% by mass, preferably 5 to 60% by mass, and more preferably 10 to 50% by mass, in view of sufficient adhesive force as an adhesive patch and of skin irritancy upon peeling.

In the case where the present invention is an adhesive patch, the adhesive layer may contain an absorption enhancer, and the absorption enhancer that can be used may be any of those compounds conventionally acknowledged to have an absorption promoting action in the skin. Examples thereof include fatty acids having chains with 6 to 20 carbon atoms, aliphatic alcohols, fatty acid esters, amides or ethers, aromatic organic acids, aromatic alcohols, aromatic organic acid esters or ethers (these may be saturated or unsaturated, and may also be cyclic, linear or branched), as well as lactic acid esters, acetic acid esters, monoterpene-based compounds, sesquiterpene-based compounds, Azone, Azone derivatives, pyrothiodecane, glycerin fatty acid esters, propylene glycol fatty acid esters, sorbitan fatty acid esters (Span series), polysorbates (Tween series), polyethylene glycol fatty acid esters, polyoxyethylene hydrogenated castor oil family (HCO series), polyoxyethylene alkyl ethers, sucrose fatty acid esters, plant oils and the like.

Specifically, caprylic acid, capric acid, caproic acid, lauric acid, myristic acid, palmitic acid, stearic acid, isostearic acid, oleic acid, linolic acid, linoleic acid, lauryl alcohol, myristyl alcohol, oleyl alcohol, isostearyl alcohol, cetyl alcohol, methyl laurate, hexyl laurate, lauric acid diethanolamide, isopropyl myristate, myristyl myristate, octyldodecyl myristate, cetyl palmitate, salicylic acid, methyl salicylate, ethylene glycol salicylate, cinnamic acid, methyl cinnamate, cresol, cetyl lactate, lauryl lactate, ethyl acetate, propyl acetate, geraniol, thymol, eugenol, terpineol, 1-menthol, borneol, d-limonene, isoeugenol, isoborneol, nerol, dl-camphor, glycerin monocaprylate, glycerin monocaprate, glycerin monolaurate, glycerin monooleate, sorbitan monolaurate, sucrose monolaurate, polysorbate 20, propylene glycol, propylene glycol monolaurate, polyethylene glycol monolaurate, polyethylene glycol monostearate, polyoxyethylene lauryl ether, HCO-60, pyrothiodecane, and olive oil are preferred, and particularly, oleic acid, oleyl alcohol, lauryl alcohol, isostearyl alcohol, lauric acid diethanolamide, glycerin monocaprylate, glycerin monocaprate, glycerin monooleate, sorbitan monolaurate, propylene glycol monolaurate, polyoxyethylene lauryl ether, and pyrothiodecane are preferred. These absorption enhancers may also be used as mixtures of two or more kinds.

In the case of adding an absorption enhancer, the additive amount is preferably 0.01 to 20% by mass, more preferably 0.05 to 10% by mass, and particularly preferably 0.1 to 5% by mass, based on the weight of the adhesive layer, in view of sufficient permeability as an adhesive preparation and of skin irritancy such as rashes and edema.

According to the present invention, if the drug is in the form of a chemically acceptable acid addition salt, it is desirable that a basic compound be included in the adhesive layer, and examples of the basic compound that may be used include low molecular weight compounds containing basic nitrogen (for example, triethanolamine, diisopropanolamine, diethanolamine, and the like), polymer compounds containing basic nitrogen (for example, aminoalkyl methacrylate copolymer E, polyvinylacetal diethylaminoacetate, polyvinylpyrridine and the like), basic alkali metal salts (sodium acetate, potassium acetate, sodium borate, sodium carbonate, trisodium citrate, sodium silicate and the like), sodium hydroxide, and potassium hydroxide. Among them, triethanolamine, diisopropanolamine, diethanolamine, aminoalkyl methacrylate copolymer E, polyvinylacetal diethylaminoacetate, sodium acetate, sodium silicate, and sodium hydroxide are preferred, and in particular, triethanolamine, aminoalkyl methacrylate copolymer E, sodium acetate and sodium hydroxide are preferred. These compounds can enhance the skin permeability of donepezil derivatives by acting on the chemically acceptable acid addition salts of donepezil to thus generate molecular and/or ion pair compounds of donepezil during the process for production of the preparation. As for the solvent that may be used in that case, production solvents such as lower alcohols, toluene, ethyl acetate, hexane and cyclohexane, compounds used as plasticizers in preparations, and the like can be used, and methanol, ethanol, isopropanol, toluene, ethyl acetate, cyclohexane, liquid paraffin, liquid polybutene and isopropyl myristate are preferred, with methanol, ethanol, isopropyl myristate and liquid paraffin being particularly preferred.

In the case where the present invention is an adhesive patch, the support layer is not particularly limited as long as it is suitable for supporting the adhesive layer, but elastic or non-elastic materials can be used. For example, woven clothes, non-woven clothes, polyurethane, polyester, polyvinyl acetate, polyvinylidene chloride, polyethylene, polyethylene terephthalate, aluminum sheet and the like, or composite materials thereof can be used.

A donepezil-containing transdermal preparation having a constitution as described above can be produced according to any conventionally known method. For example, an adhesive base containing the drug is heated to melt and is coated on a release paper or a support, and then is put together with a support or a release paper to obtain the subject preparation. Furthermore, an adhesive base component containing the drug is dissolved in a solvent such as toluene, hexane or ethyl acetate, and the solution is spread on a release paper or a support to remove the solvent by drying, and then is put together with a support or a release paper, and thus the subject preparation can be obtained.

As specific examples of well known harmful side effects caused by oral administration of donepezil, there may be mentioned in particular digestive symptoms such as anorexia and nausea, liver disorder such as increased GOT, central and peripheral nervous system symptoms such as fugue and tremor, and the like. However, the donepezil-containing transdermal preparation and a method for administration thereof according to the present invention reduce the incidence and/or the severity of harmful side effects associated with oral administration of donepezil,while enabling the sufficient amount of donepezil to be administered, so as to provide beneficial treatment.

It is contemplated that reduction of harmful side effects is partially caused by a decrease in the plasma concentration of donepezil metabolites such as 6-O-desmethyldonepezil, resulting from the donepezil-containing transdermal preparation and a method for administration thereof according to the present invention. Moreover, the method for administration makes it possible to significantly reduce the harmful side effects developed by oral administration, by controlling the absorption rate of a donepezil derivative when transporting the donepezil transdermally into the body, and the lag time taken until donepezil is absorbed (the time taken until the detected plasma concentration reaches a certain threshold value).

In the case of donepezil hydrochloride, it is known that the substance is metabolized in the body into metabolites such as 6-O-desmethyldonepezil, 5-O-desmethyldonepezil and donepezil-cis-N-oxide.

Oral administration of donepezil hydrochloride generates about 25 to 35%, based on the parent drug, of donepezil metabolites such as 6-O-desmethyldonepezil, and the amounts of generation of 5-O-desmethyldonepezil and donepezil-cis-N-oxide by oral administration are reported to be approximately 23 and 28%, respectively, based on the parent drug donepezil (Tiseo P.J., et al., Br. J. Clin. Pharmacol., Vol. 46, pp. 19-24 (1998)).

On the other hand, when the transdermal preparation of the present invention is administered, the generation of metabolites such as 6-O-desmethyldonepezil, 5-O-desmethyldonepezil or donepezil-cis-N-oxide can be kept significantly low. For example, when the transdermal preparation of the present invention is administered, the amounts of generation of metabolites in the plasma for the three kinds of 6-O-desmethyldonepezil, 5-O-desmethyldonepezil and donepezil-cis-N-oxide are all less than 5%, based on the parent drug donepezil, , and in most of cases, the amounts are less than 1 %. In this way, the transdermal preparation of the present invention can suppress the plasma concentrations of donepezil metabolites to low concentrations, which enables the reduction of harmful side effects.

Furthermore, the transdermal preparation of the present invention is a preparation which gives, when applied to human being, a value of less than 50 ng/mL·hr as the AUC for up to 12 hours after administration. In particular, it is a preparation which can give a value of less than 30 ng/mL·hr, or less than 10 ng/mL·hr, as the AUC for up to 12 hours after administration. As a result, side effects such as anorexia or vomiting caused by transdermal administration of donepezil can be effectively suppressed.

The transdermal preparation of the present invention is also a preparation which gives a value of 3 hours or more, 5 hours or more, or 10 hours, as the lag time in the process of transdermal absorption of donepezil and/or a salt thereof. When the lag time in the process of transdermal absorption of donepezil and/or a salt thereof is less than 3 hours, there is a risk of rapid rise of the blood concentration, and therefore, the transdermal preparation of the present invention makes it possible to reduce side effects by suppressing the rate of the rise.

The transdermal preparation of the present invention is also a preparation which gives a value of 1.5 or less, preferably 1.2 or less, as the ratio of the maximum plasma concentration (A) and the minimum plasma concentration (B) (A/B, peak-trough ratio) obtained after administration of the said preparation. As a result, the transient increase in the blood concentration is remarkably improved, and thus side effects can be reduced.

Furthermore, the transdermal preparation of the present invention is a preparation which gives a value of 1000 µg/hr or less, and particularly 500 µg/hr or less, as the maximum absorption rate of donepezil and/or a salt thereof. In this maximum absorption rate, the side effects considered to be caused by donepezil are reduced.

### BRIEF DESCRIPTION OF THE DRAWINGS

Fig. 1 is a schematic diagram showing a structure of an adhesive patch according to an embodiment of a transdermal preparation of the present invention;
Fig. 2 shows an X-ray diffraction pattern of donepezil hydrochloride in a preparation of Example 1;
Fig. 3 shows an X-ray diffraction pattern of donepezil hydrochloride in a preparation of Example 3;
Fig. 4 shows an X-ray diffraction pattern of a bulk powder of donepezil base;
Fig. 5 is a graph showing results of a skin permeability test on preparations of Examples 1, 5 and 7. The horizontal axis represents time, and the vertical axis represents the cumulative permeation amount of donepezil;
Fig. 6 is a graph showing results of a skin permeability test on preparations of Examples 2, 3 and 10. The horizontal axis represents time, and the vertical axis represents the cumulative permeation amount of donepezil;
Fig. 7 is a graph showing results of a skin permeability test on preparations of Examples 8 and 9. The horizontal axis represents time, and the vertical axis represents the cumulative permeation amount of donepezil; and
Fig. 8 is a diagram showing a blood profile of donepezil metabolites obtained when the preparation produced in Example 2 is used.

### EXAMPLES

Hereinafter, the present invention will be described in detail by showing Examples of the present invention, but the present invention is not intended to be limited to these Examples, and various alterations are possible within the scope of not departing from the technical idea of the present invention. Further, in the Examples, "%" in all cases is defined to mean % by mass.

### <Example 1> Formulation of SIS - pyrothiodecane (HPE)-101

| | |
|---|---|
| SIS | 14.8% |
| Polyisobutylene (Vistanex L-100) | 1.6% |
| Polyisobutylene (Opanol B-12) | 4.7% |
| Aluminum silicate | 0.4% |
| Alicyclic saturated hydrocarbon resin (Arkon P-100) | 35.9% |
| Liquid paraffin | 25.3% |
| Donepezil hydrochloride | 9.0% |
| Pyrothiodecane | 3.0% |
| Sodium acetate | 5.3% |
| Total amount | 100.0% |

### Production method:

Donepezil hydrochloride, sodium acetate, pyrothiodecane and liquid paraffin were thoroughly pulverized and mixed, and then to this mixture, SIS, Vistanex L-100, Opanol B-12, aluminum silicate, Arkon P-100 and toluene were added. The resulting mixture was stirred to obtain a solution. The solution thus obtained was degassed by a negative pressure treatment, and subsequently coated on a release liner, and the solvent was removed by drying under the conditions of 70°C for 15 minutes. This was bonded to a supporting film (Scotchpak 9732) to obtain an adhesive patch. The content of donepezil in type B crystal form in the adhesive layer of the donepezil hydrochloride adhesive patch was 7.6%.

### <Example 2> Formulation of SIS - oleic acid (1)

| | |
|---|---|
| SIS | 14.1% |
| Polyisobutylene (Vistanex L-100) | 1.5% |
| Polyisobutylene (Opanol B-12) | 4.5% |
| Aluminum silicate | 0.4% |
| Alicyclic saturated hydrocarbon resin (Arkon P-100) | 34.1 % |
| Liquid paraffin | 24.1 % |
| Donepezil hydrochloride | 9.0% |
| Oleic acid | 7.0% |
| Sodium acetate | 5.3% |
| Total amount | 100.0% |

### Production method:

Donepezil hydrochloride, sodium acetate and liquid paraffin were thoroughly pulverized and mixed, and then to this mixture, SIS, Vistanex L-100, Opanol B-12, aluminum silicate, oleic acid, Arkon P-100 and toluene were added. The resulting mixture was stirred to obtain a solution. For the rest of the procedure, the same method as in Example 1 was carried out to obtain an adhesive patch. The content of donepezil in type B crystal form in the adhesive layer was 7.6%.

### <Example 3> Formulation of SIS - oleic acid (2)

| | |
|---|---|
| SIS | 14.8% |
| Polyisobutylene (Vistanex L-100) | 1.6% |
| Polyisobutylene (Opanol B-12) | 4.7% |
| Aluminum silicate | 0.4% |
| Alicyclic saturated hydrocarbon resin (Arkon P-100) | 35.9% |
| Liquid paraffin | 25.3% |
| Donepezil hydrochloride | 9.0% |
| Oleic acid | 3.0% |
| Sodium acetate | 5.3% |
| Total amount | 100.0% |

### Production method:

An adhesive patch was obtained in the same manner as in Example 2. The content of donepezil in type B crystal form in the adhesive layer was 7.6%.

### <Example 4> Acrylic formulation - oleic acid

| | |
|---|---|
| Acrylic polymer (Duro-Tak 87-2516) | 64.0% |
| Donepezil base | 30.0% |
| Palmitic acid | 3.0% |
| Oleic acid | 3.0% |
| Total amount | 100.0% |

### Production method:

Donepezil base, palmitic acid and oleic acid were weighed, and to this mixture, an appropriate amount of methanol was added and mixed. Acrylic polymer Duro-Tak 87-2516 was added to the resulting mixture and mixed to obtain an adhesive solution. For the rest of the procedure, the same method as in Example 1 was carried out to obtain an adhesive patch. The content of donepezil in type B crystal form in the adhesive layer was 20%.

### <Example 5> Acrylic formulation - palmitic acid

| | |
|---|---|
| Acrylic polymer (Duro-Tak 87-2516) | 67.0% |
| Donepezil base | 30.0% |
| Palmitic acid | 3.0% |
| Total amount | 100.0% |

### Production method:

Donepezil base and palmitic acid were weighed, and to this mixture, an appropriate amount of methanol was added and mixed. For the rest of the procedure, the same method as in Example 4 was carried out to obtain an adhesive patch. The content of donepezil in type B crystal form in the adhesive layer was 20%.

### <Example 6> Acrylic formulation - polybutene prescription

| | |
|---|---|
| Acrylic polymer (Duro-Tak 87-2516) | 63.5% |
| Polybutene | 10.0% |
| Donepezil base | 20.0% |
| Dibutylhydroxytoluene | 0.5% |
| Palmitic acid | 3.0% |
| Cetyl palmitate | 3.0% |
| Total amount | 100.0% |

### Production method:

Donepezil base, dibutylhydroxytoluene, palmitic acid and cetyl palmitate were weighted, and to this mixture, an appropriate amount of methanol was added and mixed. Duro-Tak 87-2516 and polybutene were added to the resulting mixture and mixed to obtain an adhesive solution. For the rest of the procedure, the same method as in Example 1 was carried out to obtain an adhesive patch. The content of donepezil in type B crystal form in the adhesive layer was 10%.

### <Example 7> Acrylic formulation - acetic acid

| | |
|---|---|
| Acrylic polymer (TSR) | 67.5% |
| Acrylic polymer (Eudragit S) | 5.0% |
| Donepezil base | 20.0% |
| Acetic acid | 3.2% |
| Sodium acetate | 4.3% |
| Total amount | 100.0% |

### Production method:

Donepezil base, acetic acid and sodium acetate were weighed, and to this mixture, an appropriate amount of methanol was added and mixed. Acrylic polymer TSR and Eudragit S were added to the resulting mixture and mixed to obtain an adhesive solution. For the rest of the procedure, the same method as in Example 1 was carried out to obtain an adhesive patch. The content of donepezil in type B crystal form in the adhesive layer was 10%.

### <Example 8> Acrylic formulation - donepezil hydrochloride

| | |
|---|---|
| Acrylic polymer (Duro-Tak 87-2516) | 61.6% |
| Donepezil hydrochloride | 35.0% |
| Sodium hydroxide | 3.4% |
| Total amount | 100.0% |

### Production method:

Donepezil hydrochloride was dispersed in an appropriate amount of methanol, and then an aqueous solution of sodium hydroxide was added thereto and mixed. Duro-Tak 87-2516 was added to the resulting mixture and thoroughly mixed to obtain an adhesive solution. For the rest of the procedure, the same method as in Example 1 was carried out to obtain an adhesive patch. The content of donepezil in type B crystal form in the adhesive layer was 21.5%.

### <Example 9> Acrylic formulation - Eudragit

| | |
|---|---|
| Acrylic polymer (Duro-Tak 87-2516) | 62.1 % |
| Acrylic polymer (Eudragit S) | 5.0% |
| Donepezil hydrochloride | 30.0% |
| Sodium hydroxide | 2.9% |
| Total amount | 100.0% |

### Production method:

Donepezil hydrochloride was dispersed in an appropriate amount of methanol, and then an aqueous solution of sodium hydroxide was added thereto and mixed. Duro-Tak 87-2516 and Eudragit S were added to the resulting mixture and thoroughly mixed to obtain an adhesive solution. For the rest of the procedure, the same method as in Example 1 was carried out to obtain an adhesive patch. The content of donepezil in type B crystal form in the adhesive layer was 17.0%.

### <Example 10> Acrylic Formulation - boric acid

| | |
|---|---|
| Acrylic polymer (Duro-Tak 87-2516) | 81.5% |
| Donepezil base | 15.0% |
| Dibutylhydroxytoluene | 0.5% |
| Palmitic acid | 1.5% |
| Lauric acid diethanolamide | 0.5% |
| Boric acid | 1.0% |
| Total amount | 100.0% |

### Production method:

A donepezil base, dibutylhydroxytoluene, palmitic acid, lauric acid diethanolamide and boric acid/methanol solution was weighed, and an appropriate amount of methanol was added to the solution and mixed. Duro-Tak 87-2516 was added to the resulting mixture and mixed to obtain an adhesive solution. For the rest of the procedure, the same method as in Example 1 was carried out to obtain an adhesive patch. The content of donepezil in type B crystal form in the adhesive layer was 5%.

### <Test Example 1>

X-ray diffraction measurement was performed for donepezil hydrochloride of Examples 1 and 3 and for donepezil base of Examples. Double-sided adhesive tape was adhered to a non-reflective plate, and this was bonded with the support surface of each of the preparations produced in Examples 1 and 3. Then, the release liner was removed to expose the adhesive layer, and the resultant was taken as a sample. On the dent of a glass plate for measurement, an appropriate amount of donepezil base was taken and the surface for measurement was trimmed to become flat, and this was taken as a sample. X-ray diffraction measurement was performed using the measurement instrument and measurement conditions as shown below.

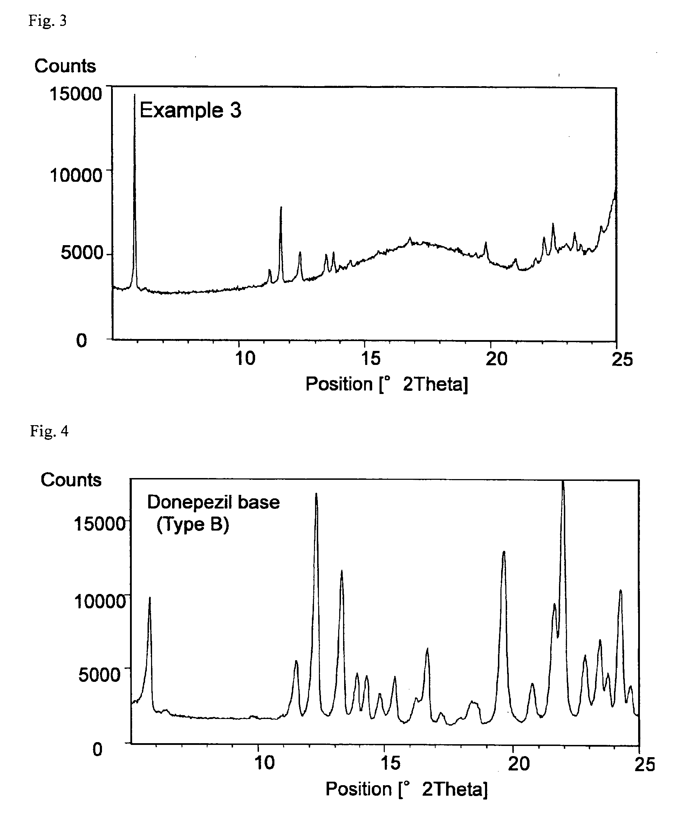

Name of instrument: X'Pert-PRO MPD (manufactured by PANalytical B.V.)
X-ray: CuKα
Scan angle (°): 5 to 50
Step size (°): 0.0167
Time per step (s): 10.160
As a result, it was confirmed that type B crystals (Position 10-15) were present in an amount corresponding to 94% of the donepezil hydrochloride incorporated into the adhesive layer in Examples 1 and 3, and type B crystals (Position 10-15) were present in an amount corresponding to 33% of the donepezil base incorporated into the adhesive layer in Examples (Figs. 2 to 4).

### <Test Example 2>

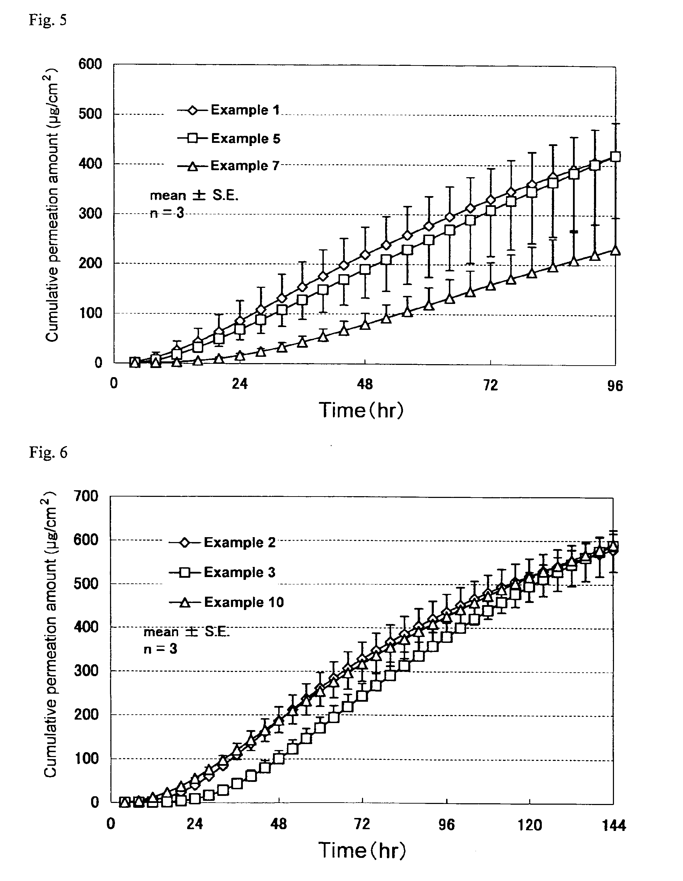

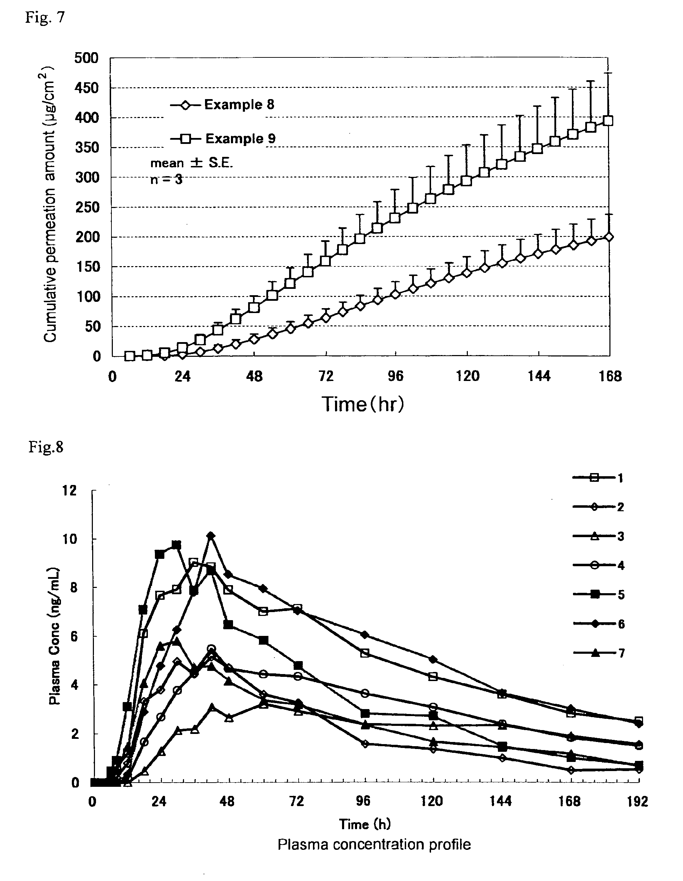

A human skin permeability test was performed on Examples 1, 3, 5 and 7 to 10. A test preparation (about 5 cm²) was adhered to the stratum corneum side of a human skin piece excised from corpse and dermatomed to about 500 µm. This assembly was mounted on a flow-through cell in which warm water at 32°C was circulated around the peripheral part, with the dermis side of the skin being disposed on the receptor solution. Phosphate buffered physiological saline (pH 7.4) was used in the receptor solution, and sampling was performed in every 4 hours or in every 6 hours up to a predetermined time, at a flow rate of about 4 mL/hr or 2 mL/hr. The flow amount of the obtained receptor solution was accurately measured, and the drug concentration was measured by a high performance liquid chromatographic method, to calculate the skin permeation rate per hour. The results for Examples 1, 5 and 7 are presented in Fig. 5, the results for Examples 2, 3 and 10 are presented in Fig. 6, and the results for Examples 8 and 9 are presented in Fig. 7.

### <Test Example 3>

The blood profile of donepezil metabolites in the case of using the preparation produced in Example 2 was examined. The preparation of Example 2 was cut out in advance in an area of 50 cm², and the preparations were adhered to 8 normal persons for 24 hours. After the adhesion, blood was sampled over time, and the concentrations of donepezil and its metabolites in the obtained plasma samples were measured using an apparatus combining a liquid chromatographic apparatus and the mass analysis method. These results are presented in Fig. 8. Furthermore, comparison with the AUC amounts of the generated metabolites (literature values) obtained in the case of oral administration of donepezil, is presented in Table 1.

**[Table 1]**

| | AUC amount of generated metabolites relative to AUC of donepezil (%) | | | |
|---|---|---|---|---|
| | 6-O-desmethyl donepezil | 5-O-desmethyl donepezil | Donepezil-cis-N-oxide | Side effects |
| Oral preparation | 35.0 | 23.0 | 28.0 | |
| Transdermal preparation | 0.1 | 0.2 | 2.9 | Absent in all of 7 Examples |

### <Test Example 4>

The absorption rate of donepezil, lag time, and the correlation between the AUC for up to 12 hours and side effects in the case of using the adhesive patch produced in Example 2 were examined according to a deconvolution method. A donepezil-containing adhesive patch was adhered, and the amount of donepezil absorbed from the donepezil-containing adhesive patch of each testee was calculated from the blood concentration profile and in vivo pharmacokinetic parameters obtained from each testee, using the deconvolution method, and then the absorption rate and lag time were also calculated. These results are presented in Table 2.

**[Table 2]**

| Testee | Maximum drug absorption rate after application | Lag time in the process of drug absorption | AUC for up to 12 hours after application | Side effects (vomiting) |
|---|---|---|---|---|
| | (µg/hr) | (hr) | (ng/mL.hr) | |
| 1 | 339 | 10.4 | 4.6 | Absent |
| 2 | 177 | 10.1 | 2.2 | Absent |
| 3 | 69.3 | 16.2 | 0.0 | Absent |
| 4 | 94.5 | 11.2 | 0.4 | Absent |
| 5 | 295 | 7.50 | 9.9 | Absent |
| 6 | 169 | 11.4 | 0.7 | Absent |
| 7 | 188 | 8.68 | 4.8 | Absent |

As is obvious from the above results, the donepezil-containing transdermal preparations obtained in the Examples of the present invention and the method of administration thereof can reduce side effects associated with the metabolites, because the amount of metabolite generation is remarkably low compared to conventional oral preparations. Furthermore, by controlling the absorption rate, lag time and the initial AUC, reduction of further harmful side effects can be achieved. Furthermore, since crystalline donepezil is dispersed in the base, the sustained release effect is enhanced, and thus reduction of harmful side effects is expected.

## Claims

1. A transdermal preparation which comprises donepezil including crystalline donepezil having type-B crystal polymorphism and/or a salt thereof in an amount of 9% by mass to 50% by mass based on the total weight of an adhesive.

2. The transdermal preparation according to claim 1, **characterized in that** the donepezil including crystalline donepezil having type-B crystal polymorphism and/or a chemically acceptable salt thereof is donepezil base.

3. The transdermal preparation according to claim 1 or 2, wherein at least 30% or more of the donepezil and/or salt thereof contained in an adhesive layer is crystalline donepezil having type-B crystal polymorphism.

4. The transdermal preparation according to any one of claims 1 to 3, wherein the preparation does not essentially contain water.

5. The transdermal preparation according to any one of claims 1 to 4, which comprises at least one adhesive selected from an acrylic polymer and a rubber-based polymer in the adhesive.

6. The transdermal preparation according to claim 5, wherein the acrylic polymer is a copolymer containing 2-ethylhexyl acrylate.

7. The transdermal preparation according to claim 5, wherein the rubber-based polymer is one or more selected from the group consisting of polyisobutylene, styrene-isoprene-styrene block copolymers and silicone adhesives.

8. The transdermal preparation according to any one of claims 1 to 5, wherein the ratio of the maximum plasma concentration (A) and the minimum plasma concentration (B) (A/B, peak-trough ratio) of donepezil and/or a chemically acceptable salt thereof after administration is 1.5 or less.

9. The transdermal preparation according to claims 1 to 5, yielding at least one or more of 5-O-desmethyldonepezil, 6-O-desmethyldonepezil and donepezil-cis-N-oxide, as the metabolite of donepezil and/or a chemically acceptable salt thereof in the plasma after administration.

10. The transdermal preparation according to claim 9, wherein the amount of generation in the plasma for one kind of the metabolite is less than 5% of the amount of donepezil and/or a chemically acceptable salt thereof contained in the transdermal preparation.

11. The transdermal preparation according to claim 9 or 10, wherein the AUC of the metabolite of donepezil after the administration of donepezil and/or a chemically acceptable salt thereof is 20% or less of the AUC of donepezil and/or a chemically acceptable salt thereof.

12. The transdermal preparation according to claim 11, wherein the AUC of the metabolite of donepezil for up to 12 hours after administration is less than 50 ng/mL·hr.

13. The transdermal preparation according to any one of claims 1 to 5, wherein the lag time associated with the absorption of donepezil and/or a chemically acceptable salt thereof is 3 hours or longer.

14. A method for administering the transdermal preparation according to any one of claims 1 to 5, wherein the maximum absorption rate of donepezil and/or a chemically acceptable salt thereof is less than 1000 µg/hr.
